# EUROPÄISCHE PATENTSCHRIFT

(11) **EP 0 498 260 B1**
(45) Veröffentlichungstag und Bekanntmachung des Hinweises auf die Patenterteilung: **13.09.1995**
(21) Anmeldenummer: 92101318.1
(22) Anmeldetag: 28.01.1992
(51) Int. Cl.: C12Q 1/34, C12Q 1/26, C12Q 1/28, G01N 33/52, C12N 9/86

(54) **Verfahren zur Stabilisierung von 1-Methylhydantoinase, Verwendung einer stabilisierten 1-Methylhydantoinase zur Bestimmung eines Analyten, entsprechendes Bestimmungsverfahren sowie hierfür geeignete Mittel**
Procedure for stabilization of 1-methylhydantoinase, use of a stabilized 1-methylhydantoinase for the determination of an analyte, corresponding determination procedure, and useful means therefor
Procédé pour la stabilisation de 1-méthylhydantoinase, usage d'un 1-méthylhydantoinase stabilisée pour la détermination d'un analyte, procédé de correspondante pour la détermination et moyens utiles à cette effet

(30) Priorität: 02.02.1991 DE 4103220
(43) Veröffentlichungstag der Anmeldung: 12.08.1992
(73) Patentinhaber: BOEHRINGER MANNHEIM GMBH, 68298 Mannheim (DE)
(72) Erfinder: Nagel, Rolf, W-6842 Bürstadt (DE); Deneke, Ulfert, Dr. rer. nat., W-6149 Rimbach-Zotzenbach (DE); Mistele, Jürgen, W-6800 Mannheim 31 (DE)

(56) Entgegenhaltungen:
- EP-A- 0 112 571
- EP-A- 0 154 269
- EP-A- 0 262 445
- ANALYTICAL LETTERS, Band 21, Nr. 6, Juni 1988, New York-Basel; J. SIEDEL et al., Seiten 1009-1017/
- BIOCHEMICAL & BIOPHYSICAL RESEARCH COMMUNICATIONS, Band 142, Nr. 3, 13. Februar 1987, New York, NY (US); JONG MIN KIM et al., Seiten 1006-1012/
- T.E. BARMAN, "Enzyme Handbook, vol. II", Springer-Verlag, Berlin-Heidelberg-New York, Abschnitt ATPase; Seiten 689-690/

## Beschreibung

Die Erfindung betrifft ein Verfahren zur Stabilisierung von 1-Methylhydantoinase, die Verwendung einer stabilisierten 1-Methylhydantoinase zur Bestimmung eines Analyten wie z.B. Creatinin, ein entsprechendes Bestimmungsverfahren sowie hierfür geeignete Mittel.

In der analytischen Chemie, insbesondere der klinisch-chemischen Diagnostik besteht ein ständig steigender Bedarf an enzymatischen Verfahren zur Bestimmung von Naturstoffen, biologischen Stoffwechselprodukten und daraus abgeleiteten Verbindungen. Die Gründe sind die außerordentlich hohe Spezifität enzymkatalytischer Substanz-Umsetzungen, ihr rascher, definierter und verlustfreier Ablauf unter milden Reaktionsbedingungen - üblicherweise zwischen 15 und 40° C im wässrigen Milieu im neutralen pH-Bereich -, sowie die Möglichkeit, sie auf einfache und empfindliche Weise, insbesondere mit photometrischen Meßmethoden entweder direkt oder mit Hilfe gekoppelter Indikatorreaktionen quantitativ erfassen zu können.

Ein enzymatisches Bestimmungsverfahren ist auch besonders wertvoll für die Bestimmung von Creatinin, einem für die Nierendiagnostik bedeutsamen Serum- und Urinbestandteil, der sich in einer seit längerem bekannten Enzymreaktion mittels der Creatinin-Iminohydrolase (E.C. 3.5.4.21) in 1-Methylhydantoin (N-Methylhydantoin, NMH), und Ammoniak umwandeln läßt.

Für die enzymatische Bestimmung von Creatinin im Serum oder Urin sind zwar schon mehrere Verfahren bekannt (Wahlefeld, A.W.; G. Holz und H.U. Bergmeyer, in H.U. Bergmeyer: Methoden der enzymatischen Analyse, 3. Auflage, Band II, Verlag Chemie, Weinheim 1974, S. 1834 - 1838; Fossati, P.; L. Prencipe, and G. Berti, Clinical Chemistry 29, 1494 - 1496 (1983); Tanganelli, E.; L. Prencipe; D. Bassi; S. Cambiaghi, and E. Murador, Clinical Chemistry 28, 1461 - 1464 (1983); sie weisen jedoch allesamt den Nachteil auf, daß sie entweder über Creatin (Wahlefeld et al.; Fossati et al.) oder Ammoniak (Tanganelli et al.) als Zwischenstufen der Reaktionssequenz verlaufen, Substanzen, die von vorneherein in wechselnden und gegenüber dem Creatinin deutlich ins Gewicht fallenden Konzentrationen in der zu analysierenden Serum- bzw. Urinprobe zugegen sind. Es sind hier also zur Creatinin-Bestimmung Differenzmessungen durch zwei getrennte oder hintereinandergeschaltete Reaktionsansätze erforderlich, einem, in dem zunächst das freie Creatin bzw. Ammoniak bestimmt wird, sowie einem zweiten, bei dem durch Zusatz entweder von Creatinin-Amidohydrolase (E.C. 3.5.2.10) oder Creatinin-Iminohydrolase (= Creatinin-Deiminase) der Anteil des zusätzlich aus Creatinin gebildeten Creatins bzw. Ammoniaks erfaßt wird ("Probe/Probenleerwert-Verfahren" oder "E₁/E₂-Verfahren").

Derartige Verfahren sind von der manuellen Durchführung her relativ aufwendig und auch nur sehr beschränkt an automatisierten Analysensystemen einsetzbar, insbesondere wenn für den vollständigen Ablauf der Umsetzungsreaktionen längere Inkubationszeiten notwendig sind. Zwar läßt sich im Prinzip durch geeignete Wahl der Reaktionsbedingungen die Creatininbestimmung mit den bekannten enzymatischen Methoden zur Umgehung der Probenleerwert-Messung im sogenannten kinetischen "fixed-time"-Verfahren durchführen; dies erfordert allerdings ein sehr genaues Einhalten der Meßzeitpunkte unter definierten Temperaturbedingungen, was mit hinreichender Präzision nur an Analysenautomaten gelingt und umgekehrt die manuelle Handhabung weitgehend ausschließt.

In der Europäischen Patentanmeldung EP-A 0 154 269 wird ein neues enzymatisches Bestimmungsverfahren für Creatinin oder 1-Methylhydantoin beschrieben. Durch die Verwendung des Enzyms 1-Methylhydantoinase (NMHase), welches zu seiner Aktivität sowohl ein Nucleosidtriphosphat, bevorzugt ATP, als auch zweiwertige Metallionen und gegebenenfalls K⁺-und/oder NH₄⁺-Ionen benötigt, wird 1-Methylhydantoin zu N-Carbamoylsarcosin hydrolysiert. Dieses Enzym ist in weiteren ebenfalls beschrieben in H. Yamada et al., FEMS Microbiol. Letters 30, 337-340 (1985), in S. Shimizu et al., Arch. Microbiol. 145, 322-328 (1986) und in Shimizu et al., Biochem. Biophys. Res. Gomm. 142, 1006-1012 (1987), und ist beispielsweise erhältlich aus Arthrobacter spec. (DSM 2563, DSM 2564) Moraxella spec. (DSM 2562), Micrococcus spec. (DSM 2565) oder Brevibacterium spec. (DSM 2843).

In einer bevorzugten Testvariante setzt die aus EP-A-0 112 571 bekannte N-Carbamoylsarcosinamidohydrolase (E.C. 3.5.3.59) N-Carbamoylsarcosin weiter zu Sarcosin um, das dann z. B. in Verbindung mit Sarcosinoxidase (E.C. 1.5.3.1) und Peroxidase (E.C. 1.11.1.7) durch einen geeigneten H₂O₂. Redoxindikator z. B. photometrisch nachgewiesen werden kann:

Creatinin + H₂O → 1-Methylhydantoin + NH₃ 1-Methylhydantoin + ATP + 2H₂O → N-Carbamoylsarcosin + ADP+Pᵢ

N-Carbamoylsarcosin + H₂O → Sarcosin + NH₃ + CO₂ Sarcosin + O₂ + H₂O → H₂O₂ + Formaldehyd + Glycin H₂O₂ + Redoxindikator (red.) → H₂O + Redoxindikator (ox.)

Ein entsprechendes photometrisches Creatininbestimmungsverfahren ist ebenfalls in J. Siedel et al., Anal. Letters 21, 1009-1017 (1988) beschrieben.

Diese Creatinin- bzw. 1-Methylhydantoinbestimmung sollte nicht von in Körperflüssigkeiten vorhandenen endogenen Substanzen gestört werden, da 1-Methylhydantoin und die Reaktionsprodukte der nachgeschalteten Indikatorreaktion keine natürlichen Bestandteile von Serum oder Urin sind. Deshalb sollte eine Probenleerwertmessung nicht nötig sein.

Es hat sich jedoch gezeigt, daß die Verwendung der NMHase für eine Creatininbestimmung durch zwei nachteilige Enzymeigenschaften eingeschränkt wird. Die NMHase benötigt für ihre Stabilität enzymgebundenes 1-Methylhydantoin (ca. 2-2,5 µmol/kU, U= internationale Einheit), sie wird also durch ihr eigenes Substrat stabilisiert. 1-Methylhydantoin bleibt im allgemeinen während der Enzymreinigung enzymgebunden. Es wird jedoch bei Anwesenheit eines Nucleosidtriphosphats und zweiwertiger Metallionen, wie z. B. Mg²⁺, in einem Bestimmungsverfahren beispielsweise für Creatinin vollständig enzymatisch abgebaut und verursacht wie ein endogenes Substrat eine Leerwertreaktion.
Diese Leerwertreaktion ist insbesondere bei einem solchen Bestimmungsverfahren besonders nachteilig, bei dem die Menge der zum Test eingesetzten NMHase größeren Schwankungen unterliegt, wie dies z. B. bei einem trägergebundenen Test der Fall sein kann. Da die Leerwertreaktion, verursacht durch enzymgebundenes NMH, einerseits abhängt von der Menge der eingesetzten NMHase, andererseits auch vom schwankenden NMH-Gehalt der NMHase selbst, muß für einen solchen Test immer separat auch die Blindreaktion bestimmt und dieser Leerwert vom Meßwert abgezogen werden.

Ohne enzymgebundenes Substrat ist die NMHase auf der anderen Seite sehr instabil und kann nicht für enzymatische Bestimmungsverfahren wie z. B. die Creatininbestimmung eingesetzt werden. Weiterhin wurde gefunden, daß das für die Enzymreaktion der NMHase notwendige Nucleosidtriphosphat in Gegenwart von NMHase nur begrenzt stabil ist, da eine ATPase-Nebenaktivität der NMHase insbesondere ATP mit der Zeit abbaut.

Aufgabe der vorliegenden Erfindung war es deshalb, ein Verfahren bereitzustellen, die NMHase so zu stabilisieren, daß auch bei Abwesenheit von enzymgebundenem 1-Methylhydantoin oder anderer Substratanaloga ein stabiles Enzym bereitgestellt wird und ein verbessertes und genaueres Bestimmungsverfahren von Creatinin oder anderer Analyte, deren Nachweis über eine durch NMHase katalysierte Reaktion durchgeführt wird, ermöglicht wird.

Diese Aufgabe wird erfindungsgemäß gelöst durch die Maßnahmen, wie sie in den Ansprüchen charakterisiert sind.

Überraschenderweise hat es sich gezeigt, daß die NMHase durch den Einsatz eines Komplexbildners, der die bei der NMHase-Reaktion eingesetzten, als Cofaktoren fungierenden zweiwertigen Metallionen komplexiert, auch bei Abwesenheit von enzymgebundenem Substrat stabilisiert werden kann.

Das erfindungsgemäße Verfahren zur Stabilisierung der NMHase ist dadurch gekennzeichnet, daß das Enzym mit einem Nucleosidtriphosphat, zweiwertigen Metallionen und einem Komplexbildner, der diese Metallionen komplexiert, behandelt wird.

Zur erfindungsgemäßen Stabilisierung der NMHase werden einer vorzugsweise gepufferten Lösung, die NMHase enthält, erst zweiwertige Metallionen und dann eine Mischung aus Nucleosidtriphosphat und soviel Komplexbildner zugegeben, daß alle anwesenden zweiwertigen Metallionen durch den Komplexbildner komplexiert werden. Alternativ kann der NMHase enthaltenden Lösung erst Nukleosidtriphosphat und dann durch Komplexbildner komplexierte zweiwertige Metallionen zugesetzt werden. Selbstverständlich kann die NMHase auch durch gleichzeitige Zugabe von zweiwertigen Metallionen, einem Komplexbildner, der die Metallionen komplexiert, und Nucleosidtriphosphat stabilisiert werden.

Unter Nucleosidtriphosphat werden insbesondere ATP und GTP verstanden, wobei ATP besonders bevorzugt ist. Zur Stabilisierung von NMHase in Lösung liegt die Konzentration des Nucleosidtriphosphats zweckmäßigerweise zwischen 0,01 und 50 mmol/l, besonders bevorzugt ist 0,1 bis 20 mmol/l, ganz besonders bevorzugt ist 1 bis 15 mmol/l.

Als zweiwertige Metallionen werden die Ionen eingesetzt, die als Cofaktoren der NMHase fungieren. Bevorzugt sind die Ionen Mg²⁺, Mn²⁺ und Zn²⁺, besonders bevorzugt sind Mg²⁺-Ionen. Zweckmäßigerweise werden sie in Form eines in wässrigem Medium löslichen Salzes, beispielweise als Chloride, Sulfate etc. in einer Konzentration zwischen 0,1 und 10 mmol/l, ganz besonders bevorzugt zwischen 0,5 und 5 mmol/l eingesetzt.

Als Komplexbildner der angesprochenen Art kommen mehrzähnige Liganden, Kryptanden, Kronenether usw. in Frage, die die eingesetzten zweiwertigen Metallionen komplexieren und mit diesen in wässrigen Lösungen stabile Chelatkomplexe bilden können. Als vorteilhaft haben sich mehrzähnige Liganden, insbesondere Nitriloessigsäure, Ethylendiamintetraessigsäure (EDTA), 1,2-Cyclohexylendinitrilotetraessigsäure, Diethylentriaminpentaessigsäure, 3-Aza-3(carboxymethyl)-pentamethylendinitrilotetraessigsäure, Zitronensäure, Ethylenbis-(oxyethylen-nitrilo)-tetraessigsäure und ähnliche als solche oder in Form in wässrigem Medium löslicher Salze erwiesen. Ganz besonders bevorzugt wird Ethylendiamintetraessigsäure (EDTA) oder Salze hiervon. Als Kationen für die in Salzform vorliegenden komplexbildenden Säuren kommen grundsätzlich alle in Frage, die zu Salzen führen, die in wässrigem Medium löslich sind und die außerdem von den Komplexbildnern nicht in vergleichbarem Maß oder stärker komplexiert werden, als die als Cofaktoren für NMHase fungierenden zweiwertigen Metallionen. Vorzugsweise werden Alkalimetallionen verwendet, besonders bevorzugt sind K⁺-Ionen.

Erfindungsgemäß wird ein Komplexbildner zur Stabilisierung der NMHase mindestens in einer solchen Konzentration eingesetzt, daß alle als Cofaktoren für NMHase fungierenden zweiwertigen Metallionen von ihm komplexiert werden. Demnach richtet sich die Konzentration des Komplexbildners nach der Konzentration der zweiwertigen Metallionen und der Zahl der Bindungsstellen und der Komplexbildungskonstante des Komplexbildners. Die notwendige Konzentration des Komplexbildners kann beispielsweise leicht durch Titration ermittelt werden.

Üblicherweise wird das Enzym in wässriger Lösung stabilisiert. Vorteilhafterweise ist die wässrige Lösung hierzu gepuffert, wobei der pH-Wert zwischen pH 7,0 und pH 9,0, bevorzugt zwischen pH 7,5 und 8,5 liegt. Zum Einstellen des pH-Wertes kommen Substanzen in Frage, die im genannten Bereich eine ausreichende Pufferkapazität aufweisen und die Aktivität des Enzyms nicht nachteilig beeinflussen, z. B. Tris, Triethanolamin oder TES [2-((Tris-(hydroxymethyl) methyl)) amino-ethansulfonsäure]. Bevorzugt ist insbesondere TES-Puffer. Die Konzentration des Puffers liegt normalerweise zwischen 50 und 500 mmol/l, ganz besonders bevorzugt zwischen 100 und 300 mmol/l.

Pro ml Pufferlösung werden normalerweise zwischen 0,01 und 40 U NMHase eingesetzt; insbesondere zwischen 0,05 und 25 U, bevorzugt 0,10 bis 20 U.
Komplexbildner wie EDTA sind aus EP-A-0 154 269 alsInhibitoren der NMHase bekannt. Deshalb war es überraschend, daß enzymgebundenes 1-Methylhydantoin in Anwesenheit eines Komplexbildners, zweiwertiger Metallionen und eines Nucleosidtriphosphats vollständig enzymatisch zu N-Carbamoylsarcosin abgebaut wird, obwohl die als Cofaktoren fungierenden zweiwertigen Metallionen vollständig durch den Komplexbildner komplexiert werden. Bei Anwesenheit weiterer Enzyme, die ihrerseits das entstandene N-Carbamoylsarcosin abbauen, wie z. B. N-Carbamoylsarcosinamidohydrolase, wird der enzymatische Abbau entsprechend fortgesetzt.

Weiter war völlig überraschend, daß das nicht mehr substratstabilisierte NMHase-Enzym in Anwesenheit des Komplexbildners über lange Zeit seine Stabilität und enzymatische Aktivität bewahrt und somit vorteilhaft in enzymatischen Bestimmungsverfahren, in denen eine stabile NMHase benötigt wird, eingesetzt werden kann.

Außerdem war überraschend, daß das eingesetzte Nucleosidtriphosphat, insbesondere ATP, nach dem Zusatz des Komplexbildners nicht mehr durch die ATPase-Nebenaktivität der NMHase abgebaut wird und so über lange Zeit stabil in Anwesenheit der NMHase gelagert werden kann. Dies ist umso unerwarteter, da man in der Literatur Angaben findet, nach denen EDTA stimulierend auf das Enzym ATPase wirkt (z. B. T.E. Barman, Enzyme Handbook, Vol. II, 1969, S. 689-690, Springer Verlag Berlin, Heidelberg, New York).

Die Lösung der erfindungsgemäß stabilisierten NMHase kann auch konzentriert, z. B. lyophilisiert werden. Auch in fester Form bleibt die NMHase stabil.

Ein weiterer Gegenstand der Erfindung ist ein Verfahren zur Bestimmung eines Analyten über eine Reaktion, die von NMHase katalysiert wird, dadurch gekennzeichnet, daß eine erfindungsgemäß stabilisierte NMHase eingesetzt wird.

Unter Analyt ist eine nachzuweisende Substanz, insbesondere in Körperflüssigkeiten, wie Blut, Serum, Plasma, Urin, Speichel etc. zu verstehen, die durch chemische oder enzymatische Reaktionen zu einem Substrat der NMHase umgesetzt und so über die NMHase-Reaktion und den nachfolgenden Nachweis der Reaktionsprodukte bestimmt werden kann. Nachzuweisender Analyt kann selbstverständlich auch NMH selbst sein.

Ein solches erfindungsgemäßes Verfahren ist z. B. ein Verfahren zur Bestimmung von Creatinin durch Überführung des Creatinins mit Creatinin-Deiminase in 1-Methylhydantoin, Hydrolyse des letzteren mit der erfindungsgemäß stabilisierten 1-Methylhydantoinase (NMHase) in Gegenwart von vorzugsweise im Überschuß vorliegenden Nucleosidtriphosphat und zweiwertigen Metallionen zu N-Carbamoylsarcosin und Nucleosiddiphosphat und Bestimmung des N-Carbamoylsarcosins, welches dadurch gekennzeichnet ist, daß vor der Durchführung der Bestimmung die NMHase mit einem Komplexbildner, der die eingesetzten zweiwertigen Metallionen komplexiert, stabilisiert wird und zum Start der Bestimmung ein Überschuß an zweiwertigen Metallionen bereitgestellt wird.

Insbesondere kann die Erfindung zur Bestimmung von Creatinin in biologischen Flüssigkeiten von Mensch und Tieren, beispielsweise Blut, Plasma, Serum, Urin, Speichel oder in flüssigen Präparationen von menschlichem oder tierischem Gewebe benutzt werden. Selbstverständlich kann die Erfindung auch zur Bestimmung von 1-Methylhydantoin selbst verwendet werden.

Bestimmungsverfahren von Creatinin über 1-Methylhydantoin und N-Carbamoylsarcosin ohne die erfindungsgemäße Stabilisierung der NMHase sind bekannt und die hierfür angegebenen und dem Fachmann bekannten Bedingungen eignen sich auch im Rahmen der Erfindung. Sie können beispielsweise der Europäischen Patentanmeldung EP-A-0 154 269 entnommen werden.

In einem bevorzugten Verfahren zur Bestimmung von Creatinin wird das nach der Hydrolyse von 1-Methylhydantoin entstehende N-Carbamoylsarcosin mit N-carbamoylsarcosinamidohydrolase unter Bildung von Sarcosin hydrolysiert. Zum Nachweis des gebildeten Sarcosins wird die Verwendung der bekannten Sarcosinoxidase-Reaktion besonders bevorzugt. Unter verschiedenen, z. B. in der EP-A-0 154 269 beschriebenen Nachweisreaktionen der entstehenden Reaktionsprodukte ist besonders der Nachweis des entstehenden H₂O₂ in einer Peroxidase-katalysierten Farbreaktion durch oxidative Kupplung von 2,4,6 Tribrom-2-hydroxybenzoesäure (TBHB) mit 4-Aminoantipyrin bevorzugt. Diese Farbreaktion kann z. B. photometrisch gemessen werden. Diese Reaktionsfolge ist im folgenden schematisch wiedergegeben: 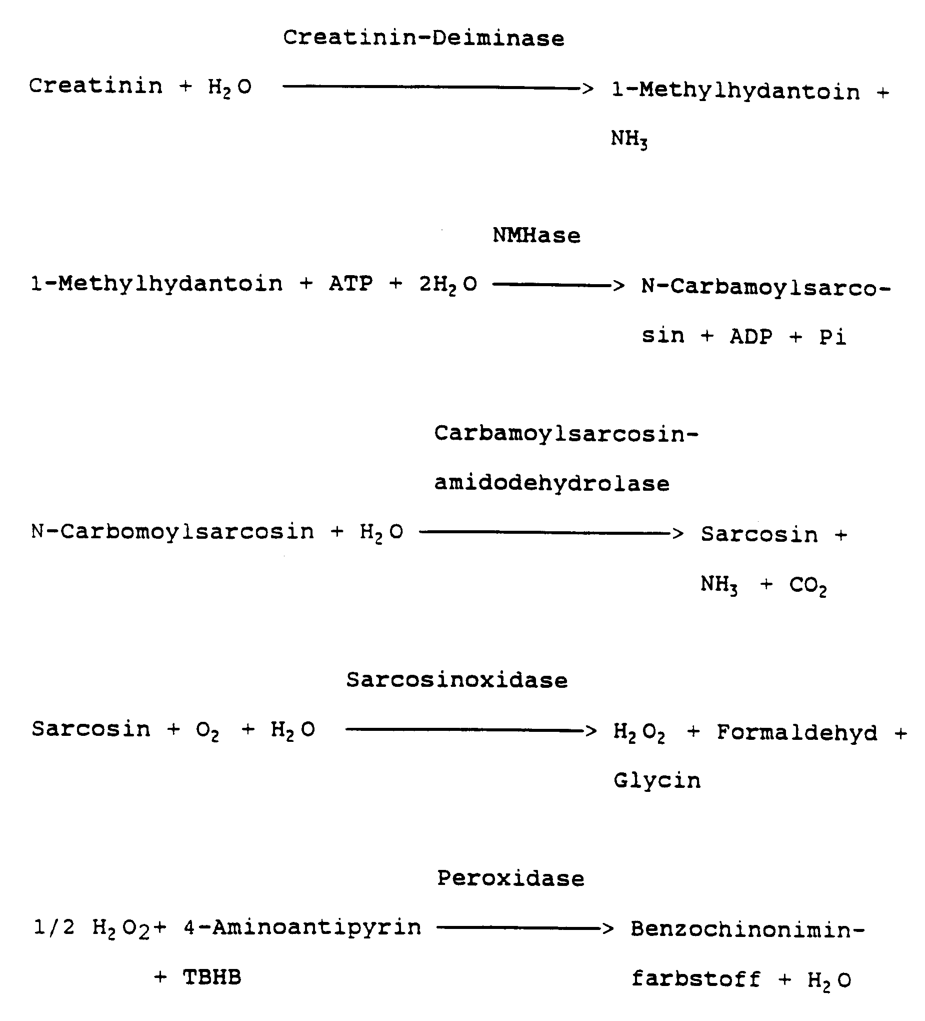
Zur Durchführung der erfindungsgemäßen Creatininbestimmung liegen die dafür nötigen Enzyme und Reagenzien in fester oder gelöster Form vor.

Der pH-Wert der wässrigen Lösung für die Analytbestimmung liegt üblicherweise zwischen pH 7,0 und pH 9,0 bevorzugt zwischen pH 7,5 und pH 8,5. Diese pH-Bedingungen sind insbesondere für die Bestimmung von Creatinin vorteilhaft geeignet. Zum Einstellen des pH-Wertes kommen beispielsweise die für die NMHase-Stabilisierung erwähnten Puffersubstanzen in Frage.

Zum Start der Meßreaktion, beispielsweise zur Bestimmung von Creatinin, werden der Reagenzlösung oder dem Reagenzgemisch, das die erfindungsgemäß stabilisierte NMHase enthält, soviel zweiwertige Metallionen zugegeben, daß die Gesamtkonzentration der Metallionen die des Komplexbildners übersteigt, d. h., daß genügend, nicht durch den Komplexbildner komplexierte, für die NMHase Reaktion notwendige zweiwertige Metallionen in der Mischung vorliegen. Dabei liegt die Gesamtkonzentration an nicht durch den Komplexbildner komplexierten zweiwertigen Metallionen im allgemeinen bei 0,1 bis 30 mmol/l, bevorzugt zwischen 0,5 und 15 mmol/l. Die Metallionen können zusammen mit dem zu bestimmenden Analyten, aber auch kurz zuvor oder danach zugegeben werden.

Als zweiwertige Metallionen eignen sich insbesondere Mg²⁺, Mn²⁺ und/oder Zn²⁺-Ionen, bevorzugt Mg²⁺-Ionen, in Form ihrer in wässrigem Medium löslichen Salzen, z. B. Chloride oder Sulfate.

Zur erfindungsgemäßen Stabilisierung von NMHase wird dieser neben anderen Substanzen auch Nucleosidtriphosphat zugesetzt. Sollte diese Menge an Nucleosidtriphosphat bei Einsatz von erfindungsgemäß stabililisierter NMHase zur Umsetzung des im Verlauf der Bestimmung eines Analyten vorliegenden NMH nicht ausreichen, muß entsprechend mehr Nucleosidtriphosphat im Bestimmungsverfahren eingesetzt werden.

Die Gesamtendkonzentration an Nucleosidtriphosphat für die Bestimmungsreaktion, insbesondere ATP und/oder GTP, liegt zweckmäßigerweise zwischen 0,01 mmol/l und 50 mmol/l, vorzugsweise zwischen 0,1 mmol/l und 20 mmol/l, besonders bevorzugt zwischen 1 mmol/l und 15 mmol/l.

Pro ml Reaktionsgemisch werden normalerweise zwischen 0,01 U und 40 U NMHase eingesetzt, insbesondere zwischen 0,05 U und 25 U, bevorzugt 0,10 bis 20 U.

Zur Aktivitätssteigerung der NMHase haben sich Zusätze von Kalium-und/oder Ammoniumsalzen, insbesondere Ammoniumsalzen als günstig erwiesen. Als Salze werden hier solche verstanden, die in wässrigem Medium löslich sind. Die Gesamtendkonzentration liegt dabei günstigerweise zwischen 0,1 mmol/l und 100 mmol/l, besonders bevorzugt zwischen 5 mmol/l und 10 mmol/l.

Die Konzentrationen der übrigen für den Creatinintest notwendigen Enzyme und Reagenzien können analog der EP-A-0 154 269 entnommen werden. Repräsentative Werte werden auch in den Beispielen der vorliegenden Anmeldung genannt.

Eine erfindungsgemäße stabilisierte NMHase ermöglicht in vorteilhafter Weise enzymatische Bestimmungsverfahren, insbesondere ein Creatininbestimmungsverfahren, bei denen 1-Methylhydantoin umgesetzt wird und bei denen stabile NMHase und stabiles Nucleosidtriphosphat Voraussetzung sind, ohne daß enzymgebundenes 1-Methylhydantoin (oder ein Substratanalogon) dieses Bestimmungsverfahren durch eine Leerwertreaktion beeinflußt.

Um eine Leerwertreaktion durch enzymgebundenes NMH bei einem erfindungsgemäßen Bestimmungsverfahren auszuschalten, wird in einem bevorzugten Verfahren die erfindungsgemäß stabilisierte NMHase vor dem Start der Meßreaktion und vor dem Zumischen des H₂0₂-Indikators mit denjenigen Enzymen in Kontakt gebracht, die für die Bestimmungsreaktion notwendig sind. Insbesondere sind dies die Enzyme N-Carbamoylsarcosinamidohydrolase, Sarcosinoxidase und gegebenenfalls Peroxidase. Diese und viele andere handelsübliche Enzyme enthalten Spuren des H₂O₂-abbauenden Enzyms Katalase. Es kann nötigenfalls auch zusätzlich zugegeben werden. Das beim enzymatischen Abbau des NMHase-gebundenen NMH gemäß obigem Reaktionsschema gebildete H₂O₂ wird so in einer Vorreaktion zur eigentlichen Meßreaktion durch Katalase beseitigt, ohne daß die langsam reagierende Katalase die Indikatorreaktion während der späteren Meßreaktion stört.

Ein weiterer Gegenstand der Erfindung ist ein Reagenz zur Durchführung der vorstehend beschriebenen erfindungsgemäßen Verfahren zur Bestimmung eines Analyten über eine von 1-Methylhydantoinase katalysierte Reaktion enthaltend 1-Methylhydantoinase, welches dadurch gekennzeichnet ist, daß es zweiwertige Metallionen, Nucleosidtriphosphat und einen Komplexbildner, der diese Metallionen komplexiert, enthält.

Ein Reagenz zum Nachweis eines Analyten, wie beispielsweise Creatinin, das NMHase enthält, ist dadurch gekennzeichnet, daß es ein oder mehrere Enzyme und Substanzen enthält, die den zu bestimmenden Analyten in NMH überführen. Für die Bestimmung von Creatinin hat sich creatinindeiminase als vorteilhaft erwiesen. Außerdem enthält das erfindungsgemäße Reagenz zweiwertige Metallionen, ein Nucleosidtriphosphat, wie ATP oder GTP, und einen Komplexbildner, der die Metallionen komplexiert, eine Puffersubstanz zur Einstellung eines pH-Wertes zwischen pH 7,0 bis 9,0 sowie ein System zum Nachweis von N-Carbamoylsarcosin. Dieses System enthält bevorzugt die Enzyme N-carbamoylsarcosinamidohydrolaSe und Sarcosinoxidase.
Zusätzlich kann dieses Nachweissystem ein Mittel zur Bestimmung von H₂O₂ enthalten. Dieses System und andere Systeme zum Nachweis der Reaktionsprodukte des Abbaus von N-Carbamoylsarcosin sind bekannt, z. B. aus H. U. Bergmeyer, Methoden der enzymat. Analyse, Verlag Chemie, Weinheim.

Gegebenenfalls kann das erfindungsgemäße Reagenz noch zusatzlich K⁺-oder NH₄⁺-Ionen, bevorzugt NH₄⁺-Ionen in Form entsprechender wasserlöslicher Salze enthalten.

Neben den genannten Bestandteilen kann das erfindungsgemäße Reagenz noch Hilfsstoffe wie beispielsweise Netzmittel, Lipasen, Gerüstbildner, Stabilisatoren usw. enthalten.

Weitere mögliche Reagenzbestandteile sowie mögliche Konzentrationsbereiche für die erfindungsgemäßen Reagenzkomponenten entsprechen den zur Durchführung des erfindungsgemäßen Verfahrens zur Bestimmung eines Analyten genannten.

Das erfindungsgemäße Reagenz kann als Lösung vorliegen. Hierbei können die erforderlichen Reagenzkomponenten gemeinsam in einer wässrigen Lösung vorhanden sein, sie können jedoch vorzugsweise auch auf mehrere Lösungen verteilt vorliegen. So ist auch Gegenstand der Erfindung ein Reagenzkit zur Bestimmung eines Analyten über eine von 1-Methylhydantoinase (NMHase) katalysierte Reaktion. Der erfindungsgemäße Kit besteht aus mindestens zwei Behältern, von denen einer erfindungsgemäß stabilisierte NMHase enthält, und ein zweiter Behälter, der zweiwertige Metallionen sowie gegebenenfalls K⁺-und/oder NH₄⁺-Ionen enthält. Ganz besonders bevorzugt enthält der erste Behälter zusätzlich die für die Bestimmungsreaktion notwendigen weiteren Enzyme und der zweite Behälter zusätzlich den für die Indikatorreaktion notwendigen Farbstoff. Die Komponenten können in wässriger Lösung gelöst vorliegen, oder sie sind in fester Form, beispielsweise als Lyophilisat vorhanden und werden vor Gebrauch gelöst. Ein solcher Reagenzkit ist besonders vorteilhaft, um die Probe mit der in dem zuerst genannten Behälter befindlichen NMHase-Zubereitung zu inkubieren und dann die Bestimmungs-Reaktion durch Zugabe der zweiwertigen Metallionen aus dem zweiten Behälter zu starten oder um die auf Analyt zu untersuchende Probe mit zweiwertigen Metallionen aus Behältnis 2 zu kombinieren und die Bestimmungs-Reaktion durch Zugabe der Enzymzubereitung aus Behältnis 1 zu starten.

Die Durchführung der erfindungsgemäßen Bestimmung von Analyten, beispielsweise Creatinin, ist auch möglich mit einem sogenannten Trockentest. Hier liegt das erfindungsgemäße Reagenz trägergebunden vor. Vorrichtungen, die zur Durchführung eines Trockentests geeignet sind, sind dem Fachmann beispielsweise aus EP-A-0 016 387, EP-A-0 309 883, DE-A-32 47 608 oder EP-A-0 262 445 bekannt. Sie werden üblicherweise als Testträger oder Teststreifen bezeichnet. Hierbei liegen die zur Durchführung eines Tests erforderlichen Reagenzien in trockener, d. h. nicht in einer Flüssigkeit gelösten Form, beispielsweise in oder auf saugfähigem Material, wie beispielsweise Papier, einem offenen Film gemäß EP-A-0 016 387, einem Glasfaservlies oder einer porösen Kunststoffmembran, um nur einige mögliche Materialien zu nennen, oder in oder auf einem quellfähigen Material, wie z. B. einem entsprechenden Kunststofffilm, Gelatine oder Cellulose, vor.

Bei Aufgabe der zu testenden Probenflüssigkeit auf den Testträger oder Eintauchen des Testträgers in die Probenflüssigkeit bildet sich in dem Testträger ein flüssiges Medium aus, innerhalb dessen die Nachweisreaktion abläuft.

Die durch die Reaktion verursachte Farbbildung kann visuell oder photometrisch, z. B. reflexionsphotometrisch ausgewertet werden. Zur Herstellung des erfindungsgemäßen Reagenzes in trägergebundener Form wird ein geeignetes Trägermaterial wie Filterpapier, Cellulose oder Kunstfaservlies mit Lösungen der erforderlichen zur Herstellung von Testträgern verwendeten Reagenzien in leicht flüchtigen Lösungsmitteln wie Wasser oder Ethanol imprägniert. Dies kann in einem Imprägnierschritt erfolgen, wobei Lösungen eingesetzt werden, die jeweils einen Teil der Bestandteile des fertigen Reagenzes enthalten. Das so behandelte Trägermaterial kann als solches verwendet werden oder in an sich bekannter Weise an Griffe oder auf steife Kunststoffolien geklebt werden. Die für die Bestimmungsreaktion notwendigen Substanzen können jedoch auch aus jeweils getrennten Lösungen in separaten Imprägniervorgängen auf oder in das Trägermaterial gebracht werden.

Statt einer mehrmaligen Imprägnierung desselben Trägermaterials können die Bestandteile des erfindungsgemäßen Reagenzes auch auf verschiedenen Trägermaterialien verteilt werden, die bei Durchführung der Analytbestimmung miteinander in einen Flüssigkeitsaustausch ermöglichenden Kontakt gebracht werden.
Zweckmäßigerweise wird die Bestimmung eines Analyten über eine durch NMHase katalysierte Reaktion mit einem mehrschichtigen Testträger durchgeführt. Dabei werden vorteilhafterweise die zur erfindungsgemäßen Stabilisierung der NMHase notwendigen zweiwertigen Metallionen, Nucleosidtriphosphat, wie ATP oder GTP, sowie der Komplexbildner, der die Metallionen komplexiert, zusammen mit NMHase und den zur Nachweisreaktion erforderlichen Enzymen auf ein als Schicht ausgebildetes Trägermaterial und die zum Teststart zusätzlich notwendige Menge an zweiwertigen Metallionen, die insgesamt einen Überschuß an Metallionen gegenüber dem eingesetzten Komplexbildner erzeugen, auf einer weiteren Schicht des gleichen oder eines anderen Trägermaterials aufgebracht. Die beiden Schichten sollen dabei in einem Flüssigkeitsaustausch ermöglichenden Kontakt stehen oder in einen solchen gebracht werden können.

In einer besonders bevorzugten Ausführungsform des Testträgers befinden sich auf der Schicht, die die zusätzliche Menge an zweiwertigen Metallionen enthält, noch die zur NMHase-Aktivitätssteigerung vorteilhaften K⁺- oder Ammoniumionen und das farbgebende Indikatorsystem.

Die Gesamtendkonzentration der einzelnen Enzyme und Reagenzien auf dem Testträger bei der Testdurchführung liegen vorteilhafterweise bei den höheren Werten der oben für ein Bestimmungsverfahren in Lösung angegebenen Konzentrationsbereiche.

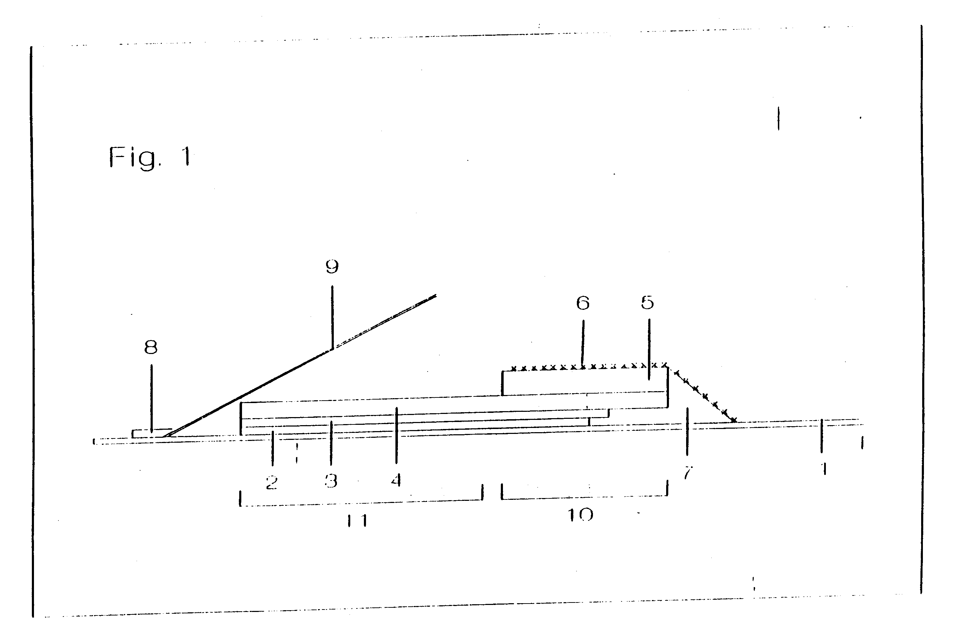

Eine ganz besonders bevorzugte Ausführungsform eines erfindungsgemäßen Testträgers zeigt Fig. 1 im Querschnitt. Eine Basisfolie (1) dient als Tragschicht für mehrere flächig anliegende flüssigkeitsabsorbierende Schichten. Auf der Basisfolie (1) sind übereinander eine oder mehrere enzymenthaltende Schichten (2), gegebenenfalls eine Indikatorschicht (3) und ein Transportvlies (4) in dieser Reihenfolge aufgebracht. Sie stehen miteinander in einem Flüssigkeitsaustausch ermöglichenden Kontakt. Das Transportvlies überdeckt vollflächig die Schichten (2) und (3). Die beiden Längsenden dieses Schichtenstapels werden als Aufgabezone (10) bzw. Detektionszone (11) bezeichnet. Auf den mit Aufgabezone (10) bezeichneten Teil des Transportvlieses (4) ist eine Erythrocytenabtrennschicht (5) aufgebracht, die von einem Abdecknetz (6) zum Schutz vor Beschädigungen überdeckt ist. Sowohl das Transportvlies (4) wie auch die Erythrocytenabtrennschicht und das Abdecknetz (6) sowie die Schichten (2) und (3) sind mit dem in der Aufgabenzone (10) liegenden Ende durch einen Schmelzkleberstreifen (7) auf der Basis (1) fixiert.

Auf der Basisfolie (1) ist eine lichtdurchlässige Klappe (9) mit einem Schmelzklebertropfen (8) so befestigt, daß sie durch externe Manipulation, z. B. Druck mit dem im Detektionsbereich liegenden Teil des Transportvlieses in Kontakt gebracht werden kann. Die Klappe (9) hat die Aufgabe, durch Druck den Flüssigkeitsaustausch innerhalb der Schichten (2), (3) und (4) zu beschleunigen. Sie besteht aus einer Folie, bevorzugt aus einem für sichtbares Licht durchlässigen Kunststoff. Besonders bevorzugt ist hierfür eine Polycarbonatfolie.

Das Transportvlies (4) kann aus einer Vielzahl von Materialien ausgewählt werden. Besonders geeignet sind Papiere, Vliese und solche Filmschichten, die aufgrund einer offenen, zahlreiche Kapillarspalten aufweisenden Struktur ein erhebliches Aufnahmevermögen für Flüssigkeit haben. Dieses Aufnahmevermögen muß so hoch sein, daß die dann absorbierte Flüssigkeitsmenge ausreicht, um sämtliche für die Nachweisreaktion erforderlichen Reagenzschichten vollständig zu benetzen. Besonders bevorzugt sind Glasfasern. Ebenfalls sind für die Erythrocytenabtrennschicht (5) Glasfasern besonders bevorzugt (vgl. DE-OS 3029579).

Die Schichten (2) bzw. (3) bestehen im allgemeinen aus mit den jeweiligen Reagenzien (Enzyme bzw. Indikatoren sowie eventuell weitere erforderliche Substanzen) imprägnierten Materialien, die saugfähige Eigenschaften haben. Hierzu sind Kunststoff- oder natürliche Materialien geeignet.

Bevorzugte mögliche Kunststoffmaterialien sind Polyamid-, ganz besonders bevorzugt Polyestergewebe.

Bevorzugte natürliche Materialien sind insbesondere Papier, ganz besonders bevorzugt ist Teebeutelpapier, wie z. B. solches mit einem Flächengewicht von etwa 10 - 20 g/m², bevorzugt 12 g/m², aus Manila Langfaser-Hanf.

Zur Durchführung der Analyse werden beispielsweise 30 µl Blut auf das Abdecknetz (6) und die Erythrocyten-Abtrennschicht (5) aufgebracht. Die Probe sickert in das darunterliegende Transportvlies (4) und die Schichten (2) und (3), wobei die Erythrocyten abgetrennt werden. Das so gebildete Plasma wird in den Schichten (2), (3) und (4) in Längsrichtung des Testträgers in den Detektionsbereich (11) transportiert. Hierbei findet in diesen Schichten eine Reaktion zwischen dem zu bestimmenden Probeninhaltsstoff und den auf die Schichten (2) und (3) imprägnierten Reagenzien statt.

Nach Ablauf einer vorgegebenen Zeit ab Probenaufgabe wird nach Herunterdrücken der lichtdurchlässigen Klappe (9) die Indikatorreaktion z. B. photometrisch bestimmt (beispielsweise durch Reflotron^{R}, Boehringer Mannheim GmbH, Mannheim, Bundesrepublik Deutschland).

Die Erfindung ist in den nachfolgenden Beispielen näher beschrieben.

### Beispiel 1

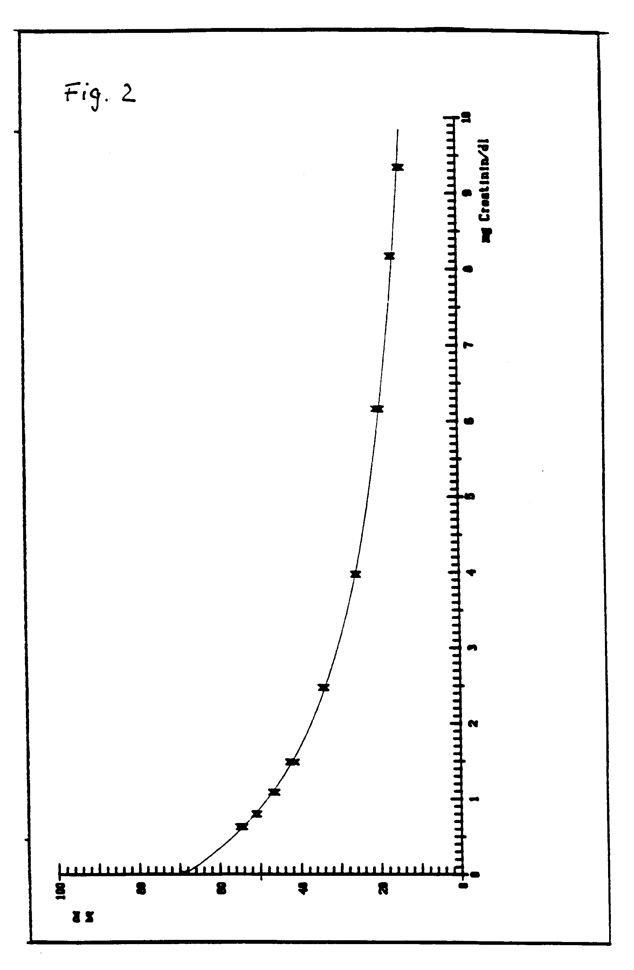

Fertigung von Reagenzträgern zum Nachweis von Creatinin mit erfindungsgemäß stabilisierter NMHase und von solchen mit NMHase gemäß Stand der Technik.
a) Reagenzträger mit NMHase nach vorhergehendem Abbau des enzymgebundenen NMH (erfindungsgemäß stabilisierte NMHase).
   Es wird ein Testträger gemäß Figur 1 hergestellt. Zur Herstellung des Enzympapiers (2) werden
   250 mmol/l TES
   12 mmol/l EDTA
   50 mmol/l ATP
   1,5 mmol/l MgCl₂
   unter pH-Wert-Einstellung mit Kaliumnydroxidlösung gelöst, End-pH-Wert 8,4. Darin werden unter Rühren nacheinander
   150 g/l Saccharose
   45 kU/l NMHase (aus Arthrobact. spec.)
   450 kU/l Creatinindeiminase (Crimi)
   186 kU/l N-Carbamoylsarcosinamidohydrolase (CSHase)
   150 kU/l Sarcosinoxidase (Sarc-OD)
   7800 kU/l Peroxidase (POD)
   150 kU/l Ascorbatoxidase (AAO)
   gelöst. Innerhalb 10 Minuten ist in dieser Lösung das enzymatisch gebundene NMH der NMHase abgebaut.
   Die gebrauchsfertige Tränklösung wird auf ein saugfähiges Papier (Langfaserpapier, Schoeller, Germsbach, Bundesrepublik Deutschland, Flächengewicht: 12g/m²) aufgebracht oder das Papier wird in die Tranklösung getaucht. Anschließend wird 5 min bei 50° C getrocknet.
   Daraus resultiert ein Enzym-Papier (2) mit folgenden Inhaltsstoffen:
   3,4 g/m² TES
   0,27 g/m² EDTA
   2,1 g/m² ATP
   0,02 g/m² MgCl₂ x 6H₂O
   9 g/m² Saccharose
   2,8 1 kU/m² NMHase
   28 kU/m² Crimi
   12 kU/m² CSHase
   9 kU/m² Sarc-OD
   474 kU/m² POD
   9 kU/m² AAO
   Analog wird das Indikatorpapier (3) hergestellt, das nach der Imprägnierung folgende Inhaltsstoffe enthält:
   0,4 g/m² 2-(3,5-Di-tert.butyl-hydroxyphenyl)-4-(5)-(9-Julolidino)-5-(4)-methyl-(1H)-imidazol. HCl (gemäß EP-A-0 161 436)
   3 g/m² Synperonic^{R}F 68 (PolyethylenglykolPolypropylenglykol-Copolymer; Serva, Heidelberg, Bundesrepublik Deutschland)
   0,8 g/m² NH₄Cl
   0,3 g/m² MgCl₂
   Zur Herstellung des Testträgers wird auf einer 500 µm dicken, 10 cm langen und 6 mm breiten Polyester-Folie (Firma ICI, Frankfurt, Bundesrepublik Deutschland) (1), das Enzympapier (2) (12 mm x 6 mm), das Indikatorpapier (3) (13 mm x 6 mm), ein 16 mm langes, 6 mm breites und 0,25 mm dickes Glasfaservlies mit einem Flächengewicht von 25 g/m² als Transportvlies (4), ein 6 mm breites, 6 mm langes und 700 µm dickes Glasfaservlies mit einem Flächengewicht von 60 g/m² als Erythrocytenabtrennvlies (5) sowie ein Schutznetz (6) aus Scrynel PE280HC rot^{R} (Polyethylen, Züricher Beuteltuchfabrik AG, Rüschlikon, Schweiz) mit den Maßen 6 x 8 mm und einer Maschenweite von 200 µm mittels eines Schmelzkleberstreifens (7) in dieser Reihenfolge wie in Figur 1 angegeben befestigt.
   Eine 200 µm dicke, 15 mm lange und 6 mm breite transparente Polycarbonatfolie (Pokalon^{R}, Lonza, Rheinfelden, Bundesrepublik Deutschland) (9) wird mittels eines Schmelzklebertropfens (8) klappenartig so auf der Trägerfolie (1) befestigt, daß sie durch Druck auf das Transportvlies (4) gepreßt werden kann.
   Die NMHase im Enzympapier ist stabil, so daß dieser Testträger mindestens 1 Jahr bei Raumtemperatur ohne merkliche Enzymaktivitätsverluste gelagert werden kann.
   Zu einer Creatininbestimmung wird auf das Schutznetz (6) 30 µl einer creatininhaltigen Probe (z. B. Blut) aufgegeben. Nach 30 sec. wird durch Druck die transparente Folie (9) auf das Transportvlies (4) die darin enthaltene Flüssigkeit und das darunterliegende Indikator- und Enzympapier (3),(2) gepreßt. Nach weiteren 60 sec. wird die Remission in Prozent bei 642 nm durch die transparente Folie (9) hindurch in einem geeigneten Reflexionsphotometer (z. B. Reflotron^{R}, Boehringer Mannheim GmbH, Mannheim, Bundesrepublik Deutschland) gemessen. Eine Eichkurve mit bekannten Creatininkonzentrationen dient zur Bestimmung unbekannter Konzentrationen (Fig. 2).
b) Reagenzträger mit MMHase ohne erfindungsgemäßen Abbau von enzymgebundenem NMH.
   Es wird eine Tränklösung für das Enzympapier gemäß Beispiel 1a gefertigt, jedoch absolut frei von Mg²⁺-Ionen. Mit dem Mg²⁺-freien Enzympapier wird ein Testträger analog Beispiel 1a gefertigt.

### Beispiel 2

Messung des Leerwertsignals der Reagenzträger gemäß Beispiel 1a (erfindungsgemäßer Testträger) und Beipiel 1b (Testträger gemäß Stand der Technik).

Auf einen Reagenzträger gemäß Beispiel 1a (enthält kein enzymgebundenes NMH) und gemäß Beispiel 1b (enthält noch enzymgebundenes NMH) werden je 30 µl einer 6 %igen Rinderserumalbuminlösung, welche weder Creatinin noch NMH enthält, aufgegeben und die Veränderung des Remissionssignals in Abhängigkeit von der Zeit dargestellt.

| Sekunden | Reagenzträger gemäß Beispiel 1a Remission % | Reagenzträger gemäß Beispiel 1b Remission % |
|---|---|---|
| 0 | 69,8 | 70,0 |
| 12 | 69,9 | 56,1 |
| 24 | 69,9 | 51,8 |
| 36 | 69,9 | 50,0 |
| 48 | 70,0 | 50,5 |
| 60 | 70,0 | 50,8 |

Nach 60 sec. hat sich ein stabiles Meßsignal eingestellt. Beim Reagenzträger gemäß Beispiel 1a bleibt das Remissionssignal praktisch unverändert, während der Reagenzträger gemäß Beispiel 1b durch den Abbau von enzymgebundenen NMH eine deutliche Reaktion anzeigt. Dieses Leerwertsignal täuscht eine Konzentration von 0,07 mmol/l Creatinin im Serum vor. Bei einer unteren Meßbereichsgrenze für Creatinin im Serum bei ca. 0,04 mmol/l verfälscht ein solches Signal die Creatininbestimmung erheblich.

Ähnlich stabile Meßsignale werden mit Reagenzträgern gemäß Beispiel 1a erhalten, bei denen zur Stabilisierung von NMHase der Komplexbildner EDTA durch verschiedene andere Komplexbildner ersetzt ist.

| Komplexbildner | Remission nach ... Sekunden | | | | | |
|---|---|---|---|---|---|---|
| | 0 | 12 | 24 | 36 | 48 | 60 |
| Nitrilotriessigsäure | 70,1 | 69,8 | 69,6 | 69,4 | 69,3 | 69,3 |
| 1,2-Cyclohexandiamin-N,N,N,N'-tetraessigsäure | 69,5, | 69,2 | 69,0 | 69,0 | 68,9 | 69,0 |
| 3-Aza-3-(carboxymethyl)-pentamethylendinitrilotetraessigsäure | 70,4 | 70,1 | 69,9 | 69,9 | 69,9 | 69,9 |
| Ethylenglycol-bis(β-aminoethylether)-N,N-tetraessigsäure | 70,3 | 70,7 | 70,6 | 70,5 | 70,5 | 70,5 |
| Citrat | 70,0 | 69,8 | 69,7 | 69,6 | 69,5 | 69,5 |
| Oxalat | 69,8 | 69,5 | 69,3 | 69,3 | 69,2 | 69,2 |

### Beispiel 3

Stabilität von NMHase
a) ohne enzymgebundenem NMH
b) mit enzymgebundenem NMH bei Anwesenheit von Mg²⁺-Ionen und ATP
c) nach erfindungsgemäßer Stabilisierung mit EDTA, Mg²⁺-Ionen und ATP

Es wurden Enzympapier-Tränklösungen gemäß Beispiel 1a hergestellt.
a) 45 U/ml NMHase ohne enzymgebundenem NMH
   ohne EDTA und MgCl₂
b) 45 U/ml NMHase mit enzymgebundenem NMH
   ohne EDTA und MgCl₂
c) 45 U/ml NMHase mit enzymgebundenem NMH erfindungsgemäße Tränklösung
   mit EDTA und MgCl₂

Die Enzymaktivität der NMHase in den drei Tränklösungen wurden in Abhängigkeit von der Zeit gemäß EP-A-0 154 269 bestimmt.

| Zeit (h) | NMHase ohne enzymgebundenes NMH, ohne EDTA, und MgCl₂ | NMHase mit enzymgebundenem NMH ohne EDTA und MgCl₂ | NMHase mit enzymgebundenem NMH in erfindungsgemäßer Tränklösung |
|---|---|---|---|
| 0 | 100 % | 100 % | 100 % |
| 0,5 | 65 | 93 | 94,7 |
| 1 | 37 | 93 | 94,5 |
| 2 | 15 | 95 | - |
| 4 | 7 | 97 | 106 |
| 6 | 1 | 90 | - |
| 24 | 0 | 76 | 97 |

Die NMHase ohne enzymgebundenes NMH zeigt danach eine drastische Instabilität, welche sie nicht mehr verarbeitbar macht. Die NMHase mit enzymgebundenem NMH ist dagegen stabil, zeigt aber in Beispiel 1b eine Leerwertreaktion. Die erfindungsgemäß behandelte NMHase zeigt dagegen eine ausgezeichnete Stabilität, obwohl sie kein enzymgebundenes NMH mehr enthält.

### Beispiel 4

Stabilität von ATP in Gegenwart von
a) erfindungsgemaß stabilisierter NMHase
b) NMHase ohne EDTA-Behandlung

Es werden 2 Enzymtränklösungen nach Beispiel 1a hergestellt
a) mit EDTA
b) ohne EDTA

und die Konzentration des ATP in Abhängigkeit von der Zeit gemessen.

| Zeit (h) | Tränklösung mit EDTA | Tränklösung ohne EDTA |
|---|---|---|
| 0 | 100 % | 100 % |
| 1,5 | 95,1 | 33 |
| 4 | 90,9 | 5 |
| 24 | 87,9 | > 1 |

ATP in der erfindungsgemäßen Tränklösung a) bleibt befriedigend stabil, während es in der Tränklösung b) ohne EDTA in kurzer Zeit abgebaut wird.

Die folgenden Werte geben die ATP-Konzentration (in %) in Tränklösungen, in denen EDTA durch verschiedene andere Komplexbildner ersetzt ist, nach 24 Stunden an. (ATP-Anfangskonzentration: 100 %).

| Komplexbildner | ATP-Konzentration in % nach 24 h |
|---|---|
| Nitrilotriessigsäure | 72,6 |
| 1,2 Cyclohexandiamin-N,N,N,N'-tetraessigsäure | 81,7 |
| 3-Aza-3-(carboxymethyl)-pentamethylendinitrilotetraessigsäure | 91,7 |
| Ethylenglycol-bis(β-aminoethylether)-N,N-tetraessigsäure | 66,5 |
| Citrat | 62,3 |
| Oxalat | 57,3 |

## Patentansprüche

1. Verfahren zur Stabilisierung von 1-Methylhydantoinase (NMHase), welche gegebenenfalls enzymgebundenes 1-Methylhydantoin enthält, dadurch gekennzeichnet, daß die NMHase mit zweiwertigen Metallionen, einem Nucleosidtriphosphat und einem Komplexbildner, der diese zweiwertigen Metallionen komplexiert, behandelt wird.

2. Verfahren nach Anspruch 1 dadurch gekennzeichnet, daß als zweiwertige Metallionen Mg²⁺-, Mn²⁺- und/oder Zn²⁺-Ionen eingesetzt werden.

3. Verfahren nach einem der Ansprüche 1 oder 2, dadurch gekennzeichnet, daß als Komplexbildner Ethylendiamintetraessigsäure oder ein Salz hiervon eingesetzt wird.

4. Verfahren nach einem der Ansprüche 1 bis 3 dadurch gekennzeichnet, daß als Nucleosidtriphosphat ATP eingesetzt wird.

5. Verfahren zur Bestimmung eines Analyten über eine von NMHase katalysierte Reaktion dadurch gekennzeichnet, daß
NMHase, die gemäß einem Verfahren nach einem der Ansprüche 1 bis 4 stabilisiert ist, eingesetzt wird,
die NMHase-Reaktion durch einen Überschuß an zweiwertigen Metallionen gegenüber dem Komplexbildner und gegebenenfalls K⁺ und/oder NH₄⁺-Ionen gestartet wird
und N-Carbamoylsarcosin über ein geeignetes Verfahren bestimmt wird.

6. Verfahren gemäß Anspruch 5 dadurch gekennzeichnet, daß N-Carbamoylsarcosin mit N-Carbamoylsarcosinamidohydrolase und Sarcosinoxidase bestimmt wird.

7. Verwendung einer mit zweiwertigen Metallionen, einem Nucleosidtriphosphat und einem Komplexbildner, der die zweiwertigen Metallionen komplexiert, stabilisierten NMHase zur Bestimmung eines Analyten über eine von NMHase katalysierte Reaktion.

8. Reagenz zur Bestimmung eines Analyten über eine von 1-Methylhydantoinase (NMHase) katalysierte Reaktion enthaltend NMHase, dadurch gekennzeichnet, daß es zweiwertige Metallionen, Nucleosidtriphosphat und einen Komplexbildner, der die zweiwertigen Metallionen komplexiert, sowie gegebenenfalls K⁺-und/oder NH₄⁺-Ionen enthält.

9. Reagenz zur Bestimmung von Creatinin enthaltend NMHase und Creatinindeiminase dadurch gekennzeichnet, daß es zweiwertige Metallionen, Nucleosidtriphosphat und einen Komplexbildner, der die zweiwertigen Metallionen komplexiert, gegebenenfalls K⁺- und/oder NH₄⁺-Ionen sowie ein System zum Nachweis von N-Carbamoylsarcosin und eine Puffersubstanz zur Einstellung eines pH-Wertes zwischen pH 7,0 und pH 9,0 enthält.

10. Reagenz gemäß Anspruch 9, dadurch gekennzeichnet, daß es als System zum Nachweis von N-Carbamoylsarcosin, N-Carbamoylsarcosinamidohydrolase, Sarcosinoxidase, Peroxidase sowie ein H₂O₂-Nachweisreagenz enthält.

11. Reagenz gemäß Anspruch 10, dadurch gekennzeichnet, daß es zusätzlich Katalase enthält.

12. Reagenz gemäß einem der Ansprüche 8 - 11, dadurch gekennzeichnet, daß es trägergebunden vorliegt.

13. Reagenzkit zur Bestimmung eines Analyten über eine von 1-Methylhydantoinase (NMHase) katalysierte Reaktion mit mindestens 2 Behältern, wobei ein Behälter NNHase, zweiwertige Metallionen, Nucleosidtriphosphat und einen Komplexbildner, der die zweiwertigen Metallionen komplexiert, sowie gegebenenfalls K⁺- und/oder NH₄⁺-Ionen enthält und ein zweiter Behälter zweiwertige Metallionen sowie gegebenenfalls K⁺- und/oder NH₄⁺-Ionen enthält.

14. Reagenzkit gemäß Anspruch 13, dadurch gekennzeichnet, daß der erste Behälter zusätzlich die Enzyme N-Carbamoylsarcosinamidohydrolase, Sarcosinoxidase und gegebenenfalls Peroxidase und der zweite Behälter zusätzlich ein H₂O₂-Nachweisreagenz enthält.

15. Mehrschichtiger Testträger zur Bestimmung eines Analyten über eine durch NMHase katalysierte Reaktion, dadurch gekennzeichnet,
daß er in einer Schicht NMHase zusammen mit zweiwertigen Metallionen, Nucleosidtriphosphat und einem Komplexbildner, der die zweiwertigen Metallionen komplexiert, eine Puffersubstanz für pH 7,0 bis 9,0, sowie gegebenenfalls die für die Bestimmungsreaktion notwendigen weiteren Enzyme, sowie in einer weiteren Schicht, die mit der NMHase enthaltenden Schicht in einem Flüssigkeitsaustausch ermöglichenden Kontakt steht oder in einen solchen gebracht werden kann, eine zusätzliche Menge zweiwertiger Metallionen, die die überschüssige Menge an Komplexbildner übersteigt, enthält.

16. Enzympräparat enthaltend NMHase, zweiwertige Metallionen, Nucleosidtriphosphat und Komplexbildner, der die zweiwertigen Metallionen zu komplexieren in der Lage ist, in gelöstem oder festen Zustand.

## Claims

1. Process for the stabilisation of 1-methylhydantoinase (NMHase) which possibly contains enzyme-bound 1-methylhydantoin, characterised in that the NMHase is treated with divalent metals ions, a nucleoside triphosphate and a complex former which complexes these divalent metal ions.

2. Process according to claim 1, characterised in that Mg²⁺, Mn²⁺ and/or Zn²⁺ are used as divalent metal ions.

3. Process according to one of claims 1 or 2, characterised in that ethylenediamine-tetraacetic acid or a salt thereof is used as complex former.

4. Process according to one of claims 1 to 3, characterised in that ATP is used as nucleoside triphosphate.

5. Process for the determination of an analyte via an NMHase-catalysed reaction, characterised in that NMHase which is stabilised according to a process according to one of claims 1 to 4 is used, the NMHase reaction is started by an excess of divalent metal ions compared with the complex former and possibly K⁺ and/or NH₄⁺ ions and N-carbamoylsarcosine is determined via a suitable process.

6. Process according to claim 5, characterised in that N-carbamoylsarcosine is determined with N-carbamoylsarcosine amidohydrolase and sarcosine oxidase.

7. Use of an NMHase stabilised with divalent metal ions, a nucleoside triphosphate and a complex former which complexes the divalent metal ions for the determination of an analyte via a reaction catalysed by NMHase.

8. Reagent for the determination of an analyte via a reaction catalysed by 1-methylhydantoinase (NMHase), containing NMHase, characterised in that it contains divalent metal ions, nucleoside triphosphste and a complex former which complexes the divalent metal ions, as well as possibly K⁺ and/or NH₄⁺ ions.

9. Reagent for the determination of creatinine, containing NMHase and creatinine deiminase, characterised in that it contains divalent metal ions, nucleoside triphosphate and a complex former which complexes the divalent metal ions, possibly K⁺ and/or NH₄⁺ ions, as well as a system for the detection of N-carbamoylsarcosine and a buffer substance for the adjustment of a pH value between pH 7.0 and pH 9.0.

10. Reagent according to claim 9, characterised in that, as system for the detection of N-carbamoylsarcosine, it contains N-carbamoylsarcosine amidohydrolase, sarcosine oxidase, peroxidase, as well as an H₂O₂ detection reagent.

11. Reagent according to claim 10, characterised in that it additionally contains catalase.

12. Reagent according to one of claims 8 - 11, characterised in that it is present carrier-bound.

13. Reagent kit for the determination of an analyte via a reaction catalysed by 1-methylhydantoinase (NMHase) with at least 2 containers, whereby one container contains NMHase, divalent metal ions, nucleoside triphosphate and a complex former which complexes the divalent metal ions, as well as possibly K⁺ and/or NH₄⁺ ions and a second container contains divalent metal ions, as well as possibly K⁺ and/or NH₄⁺ ions.

14. Reagent kit according to claim 13, characterised in that the first container additionally contains the enzymes N-carbamoylsarcosine amidohydrolase, sarcosine oxidase and possibly peroxidase and the second container additionally contains an H₂O₂ detection reagent.

15. Multilayer test carrier for the determination of an analyte via a reaction catalysed by NMHase, characterised in that, in one layer, it contains NMHase together with divalent metal ions, nucleoside triphosphate and a complex former which complexes divalent metal ions, a buffer substance for pH 7.0 to 9.0, as well as possibly the further enzymes necessary for the determination reaction, as well as a second layer which stands in contact with the NMHase-containing layer making possible a liquid exchange or can be brought into such contact, an additional amount of divalent metal ions which exceeds the excess amount of complex former.

16. Enzyme preparation containing NMHase, divalent metal ions, nucleoside triphosphate and complex former which is able to complex divalent metal ions, in dissolved or solid state.

## Revendications

1. Procédé pour la stabilisation de la 1-méthylhydantoïnase (NMHase), qui contient éventuellement de la 1-méthylhydantoïne liée à l'enzyme, caractérisé en ce que l'on traite la NMHase avec des ions métalliques divalents, un nucléosidetriphosphate et un agent complexant, qui complexe ces ions métalliques divalents.

2. Procédé selon la revendication 1, caractérisé en ce que, comme ions métalliques divalents, on utilise les ions Mg²⁺, Mn²⁺ et/ou Zn²⁺.

3. Procédé selon l'une des revendications 1 ou 2, caractérisé en ce que, comme agent complexant, un utilise l'acide éthylènediaminetétraacétique ou un de ses sels.

4. Procédé selon l'une quelconque des revendications 1 à 3, caractérisé en ce que, comme nucléosidetriphosphate, on utilise l'ATP.

5. Procédé de détermination d'un analyte, par l'intermédiaire d'une réaction catalysée par la NMHase, caractérisé en ce que :
on utilise une NMHase stabilisée selon un procédé tel que revendiqué dans l'une des revendications 1 à 4,
on amorce la réaction de la NMHase à l'aide d'un excès d'ions métalliques divalents par rapport à l'agent complexant et éventuellement d'ions K⁺ et/ou NH₄⁺
et on détermine la N-carbamoylsarcosine par un procédé approprié.

6. Procédé selon la revendication 5, caractérisé en ce que la N-carbamoylsarcosine est déterminée avec la N-carbamoylsarcosineamidohydrolase et la sarcosineoxydase.

7. Utilisation d'une NMHase stabilisée avec des ions métalliques divalents, un nucléosidetriphosphate et un agent complexant, qui complexe les ions métalliques divalents, pour la détermination d'un analyte à l'aide d'une réaction catalysée par la NMHase.

8. Réactif pour la détermination d'un analyte par l'intermédiaire d'une réaction catalysée par la 1-méthylhydantoïnase (NMHase), caractérisé en ce qu'il contient des ions métalliques divalents, un nucléosidetriphosphate et un agent complexant, qui complexe les ions métalliques divalents, ainsi qu'éventuellement des ions K⁺ et/ou NH₄⁺.

9. Réactif pour la détermination de la créatinine, contenant de la NMHase et de la créatininedésiminase, caractérisé en ce qu'il contient des ions métalliques divalents, un nucléosidetriphosphate et un agent complexant, éventuellement des ions K⁺ et/ou NH₄⁺ ainsi qu'un système pour la détection de la N-carbamoylsarcosine et une substance tampon pour l'ajustement d'un pH compris entre 7,0 et 9,0.

10. Réactif selon la revendication 9, caractérisé en ce que, comme système de détection de la N-carbamoylsarcosine, il contient de la N-carbamoylsarcosineamido hydrolase, de la sarcosineoxydase, de la peroxydase ainsi qu'un réactif de détection de H₂O₂.

11. Réactif selon la revendication 10, caractérisé en ce qu'il contient en outre de la catalase.

12. Réactif selon l'une quelconque des revendications 8-11, caractérisé en ce qu'il est lié à un support.

13. Kit de réactif pour la détermination d'un analyte par l'intermédiaire d'une réaction cataysée par la 1-méthylhydantoïnase (NMHase), comportant au moins deux récipients, l'un des récipients contenant la NMHase, des ions métalliques divalents, un nucléosidetriphosphate et un agent complexant qui complexe les ions métalliques divalents, ainsi qu'éventuellement des ions K⁺ et/ou NH₄⁺, et le deuxième récipient contenant des ions métalliques divalents ainsi qu'éventuellement des ions K⁺ et/ou NH₄⁺.

14. Kit de réactif selon la revendication 13, caractérisé en ce que le premier récipient contient en outre les enzymes N-carbamoylsarcosineamidohydrolase, sarcosine-oxydase et éventuellement peroxydase et le second récipient contient en outre un réactif de détection de H₂O₂.

15. Support de test multicouche pour la détermination d'un analyte par l'intermédiaire d'une réaction catalysée par la NMHase, caractérisé en ce qu'il contient, dans une couche, la NMHase conjointement avec des ions métalliques divalents, un nucléosidetriphosphate et un agent complexant qui complexe les ions métalliques divalents, une substance tampon pour ajuster le pH entre 7,0 et 9,0, ainsi qu'éventuellement les enzymes supplémentaires nécessaires pour la réaction de détermination, et dans une autre couche, qui est, ou peut être amenée, en contact permettant un échange de liquide avec la couche contenant la NMHase, une quantité supplémentaire dons métalliques divalents, qui est supérieure à la quantité d'agent complexant en excès.

16. Préparation enzymatique contenant la NMHase, des ions métalliques divalents, un nucléosidetriphosphate et un agent complexant, qui est capable de complexer les ions métalliques divalents, à l'état dissous ou solide.
